# EUROPÄISCHE PATENTSCHRIFT

(11) **EP 1 778 036 B1**
(45) Veröffentlichungstag und Bekanntmachung des Hinweises auf die Patenterteilung: **17.06.2009**
(21) Anmeldenummer: 05782735.4
(22) Anmeldetag: 12.08.2005
(51) Int. Cl.: A42B 3/16, A61F 11/06, H04R 1/10

(54) **GEHÖRSCHUTZ- UND/ODER LAUTSPRECHERELEMENT**
HEARING PROTECTION AND/OR LOUDSPEAKER ELEMENT
ELEMENT SERVANT DE PROTECTION AUDITIVE ET/OU DE HAUT-PARLEUR

(30) Priorität: 19.08.2004 AT 13942004
(43) Veröffentlichungstag der Anmeldung: 02.05.2007
(73) Patentinhaber: Pfanner, Anton, 6845 Hohenems (AT)
(72) Erfinder: Pfanner, Anton, 6845 Hohenems (AT)
(74) Vertreter: Hofmann, Ralf U.
(86) Internationale Anmeldenummer: PCT/EP2005/008811
(87) Internationale Veröffentlichungsnummer: WO 2006/018250

(56) Entgegenhaltungen:
- WO-A-98/56268
- DE-U1- 9 309 795
- GB-A- 623 131
- GB-A- 2 336 291
- US-A- 997 673
- US-A- 1 225 422

## Beschreibung

Die Erfindung betrifft ein Gehörschutz- und/oder Lautsprecherelement mit einem Tragteil und einem mit einer Ohrmuschel eines Benützers in Wirkverbindung bringbaren, als Gehörschutz und/oder Lautsprecher wirksamen Modul, wobei das Tragteil am Kopf des Benützers festlegbar ist, und das als Gehörschutz und/oder Lautsprecher wirksame Modul relativ zum Tragteil von einer Ruhestellung in Richtung zu einer Einsatzstellung verstellbar ist und wobei das Modul propfenartig und gegebenenfalls als ein den Muscheleingang eines menschlichen Ohres abdeckender Teil ausgebildet ist.

Es sind schon eine Vielzahl verschiedener Ausführungen von Schutzhelmen und von Befestigungssystemen zum Festlegen von Gehörschutzmuscheln an einem Arbeitshelm bekannt geworden. Bei einer bekannten Ausgestaltung (EP 0646333 B1) werden die Gehörschutzmuscheln über Hebelarme verschwenkbar am Schutzhelm gehalten, wobei diese in der Einsatzstellung, also im Bereich des Ohres des Benützers, mit entsprechendem Anpressdruck anliegen müssen, damit die Ohren gegen den auftretenden Lärm geschützt sind. Es werden hier nicht nur besonders große Gehörschutzmuscheln benötigt - da eben das ganze Ohr abgedeckt werden muss - sondern es ist dadurch auch der Nachteil gegeben, dass der Benützer einer enormen Hitzebelastung ausgesetzt ist, weil die angepressten Gehörschutzmuschein zu einer besonders intensiven Schweißbildung führen.

Weiter ist eine Ausführung eines Ohrschützers bekannt (US-A-997673), bei der das ganze Ohr abdeckende Schalen an einem bogenförmig über den Kopf des Benützers geführten Haltebügel angeordnet sind. Sowohl die das ganze Ohr abdeckenden Schalen als auch in das Ohr einführbare Pfropfen sind an dem Haltebügel verstellbar gehalten, wobei es aber einer Voreinstellung bedarf. Nach dem Ansetzen dieses Ohrschützers liegen die Schalen an den Ohren an bzw. decken den Ohrbereich ab und die Pfropfen sind in den Gehörgang eingeführt. Eine Verstellung oder Einstellung der Pfropfen gegenüber den Schalen ist bei aufgesetztem Ohrschützer nicht möglich. Außerdem muss hier immer die Ohren des Benützers zur Gänze abgedeckt sein. Die hier als Schalen ausgeführten Gehörschutzmuscheln führen also wiederum bei einer Hitzebelastung zu einer besonderen Schweißbildung.

Ferner ist ein Schutzhelm bekannt geworden (DE 3722465 A1), bei welchem eine an der Helmschale befestigte und auf der Außenseite betätigbare Pumpvorrichtung auf der Außenseite der Helmschale zugänglich ist, um zwei Blasbalge aufzublasen, welche jeweils zwischen der Helmschale und einer zugeordneten schalldämmenden Ohrenkappe angeordnet sind. Die Ohrenkappen sind an der Helmschale befestigt. Die Blasebalge können über Entlüftungsvorrichtungen mit einem auf der Außenseite der Helmschale zugänglichen Bedienungselement entlüftet werden, wenn der Schutzhelm abgenommen werden soll. Auch hier sind das ganze Ohr des Benützers abdeckende und mit einem entsprechenden Anpressdruck anliegende Ohrenkappen vorhanden, die zu Schweißbildung führen und daher oft schon aus diesem Grunde nicht zu Einsatz kommen.

Die WO 98/56268 offenbart ebenso wie die US 1,225,442 einen gattungsgemäßen Gehörschutz.

Die Erfindung hat sich daher zur Aufgabe gestellt, diesbezüglich eine Verbesserung für gattungsgemäße Gehörschutz- und/oder Lautsprecherelemente vorzuschlagen.

Erfindungsgemäß gelingt dies dadurch, dass das Modul über ein elastisch dehnbar und zusammendrückbares und/oder federnd ausgebildetes Halteelement mit dem Tragteil verbunden ist, sodass das Modul elastisch und federnd nachgiebig aus einer Einsatzstellung bei einem Gehöreingang des Benützers wegschwenkbar ist.

Durch den Gegenstand gemäß Patentanspruch 1 wird erreicht, dass das Modul schnell in die wirksame Stellung gebracht werden kann, nur ein geringes Gewicht aufweist und vor allem nur den Muscheleingang des Ohres abdeckt. Es kommt daher zu keiner Schweißbildung wegen der anliegenden Module, wobei aber trotzdem gewährleistet ist, dass der Gehörgang wirksam gegenüber einem vorhandener Lärmpegel geschützt ist.

Durch die erfindungsgemäßen Maßnahmen ist gewährleistet, dass sich das Modul auch bei einer eventuellen Bewegung des Tragteiles - z.B. zusammen mit einem Helm - nicht als starr gegenüber dem Gehörgang auswirkt und dass dadurch auch weitgehend verhindert werden kann, dans sich Verletzungen im Ohrbereich ergeben.

Die einfachste Konstruktion ist dann gegeben, wenn das Modul am Tragteil ausfahrbar und einziehbar gehalten ist. Es ist ja an sich nur eine geradlinige Verschiebung vom Tragteil in Richtung zum Ohr hin und gegebenenfalls wieder zurück notwendig, so dass die Erfindung auch verschiedene Möglichkeiten in konstruktiv einfachem Aufbau ermöglicht.

In diesem Zusammenhang ist es denkbar, dass das Modul über eine mechanische Verstelleinrichtung mit dem Tragteil verbunden ist. Dies ist die einfachste Variante und ist daher auch nicht besonders aufwendig.

Mehr Möglichkeiten bieten sich, wenn das Modul über einen elektrischen Antrieb mit dem Tragteil verbunden ist. Dann können auch verschiedene Steuerelemente mit eingesetzt werden, um eben die Variationsmöglichkeiten noch zu verbessern.

Gerade in diesem Zusammengang ist es daher durchaus möglich, dass die Verstellung des Moduls gegenüber dem Tragteil in Abhängigkeit von einem voreinstellbaren Lärmpegel und/oder einem eingeschalteten Lautsprecher erfolgt. Es ist daher denkbar, einen Lärmpegel einzustellen, der für das Gehör noch nicht gefährdend ist. Sobald dieser Lärmpegel überschritten wird, kann das Modul automatisch in seine wirksame Stellung im Bereich des Gehöreinganges vorgeschoben werden. Das gleiche wäre auch denkbar, wenn das Modul auch als Lautsprecher (Kopfhörer) im Einsatz ist. Sobald z.B. ein Anruf auf einem Handy kommt, könnte der Vorschub des als Lautsprecher ausgebildeten Moduls bewirkt werden. In gleicher Weise kann auch der Vorschub bewirkt werden, wenn der Benützer telefonieren will. Es ist dabei auch grundsätzlich möglich, in dem Lautsprecher bzw. Kopfhörer ein Mikrophon zu integrieren, so dass beim Telefonieren auch Umweltgeräusche, wie z.B. Windgeräusche ausgeschaltet werden können.

Nachdem ja ein erfindungsgemäßes Gehörschutz- und oder Lautsprecherelement für den Einsatz bei entsprechenden Benützern vorgesehen ist, wird selbstverständlich vorgesehen, dass zwei Module um 180° versetzt zueinander am gleichen Tragteil angeordnet sind.

Eine optimale Möglichkeit bietet sich dann an, wenn das Modul über von außerhalb am Tragteil angeordneten Drehknöpfen verstellbar ist. Der Benützer kann dann immer bei einem aufgesetzten Tragteil und somit auch bei einem eventuell aufgesetzten Helm das Modul vorschieben oder in die zurückgezogene Grundstellung bewegen.

Dabei liegt eine vorteilhafte Konstruktion darin, dass für den Verschiebebereich des Moduls Endanschläge vorgesehen sind. Es sind dadurch Beschädigungen an der Konstruktion selbst, aber auch am Ohr des Benützers ausgeschlossen.

Wenn das Modul oder die Verstelleinrichtung in der Vorschubstellung einrastbar ist, wobei das Modul trotzdem elastisch und/oder federnd wegschwenkbar ist, dann ist die Gewähr gegeben, dass das Modul nicht ungewollt bei normalen Erschütterungen aus seiner vorgeschobenen Schutzstellung zurückgeführt wird. Trotzdem kann es aber nicht zu Verletzungen kommen, wenn z.B. der Tragteil gewaltsam vom Kopf des Benützers gezogen oder geschoben wird.

Eine Ausführungsvariante sieht vor, dass das Halteelement als in eine kreis- oder spiralförmig verlaufende Nut im Drehknopf eingelegter elastisch abbiegbarer Stab ausgeführt ist, welcher im Vorschubbereich des Moduls in die Vorschubrichtung umgelenkt ist. Ein solcher Stab kann z.B. als Federelement aufgeführt sein, welches für eine Kraftübertragung in axialer Richtung steif ist, jedoch immer elastisch federnd abgebogen werden kann. Sowohl bei der Ausbildung als Federelement oder als Stab wird eine Art Rohr gebildet, über welches auch Kabel, wie Lautsprecher- bzw. Mikrophonleitungen, zu dem Modul zugeführt sein können.

Damit auch eine Möglichkeit der Anpassung an verschiedene Kopfgrößen oder an die verschieden Lage der Ohren eines Benützers gegeben ist, wird ferner vorgeschlagen, dass das Halteelement und somit auch das Modul und somit das gesamte Gehörschutz- und/oder Lautsprecherelement quer zur Vorschubrichtung desselben in vertikaler und/oder horizontaler Richtung verstellbar am Tragteil gehalten sind.

Um einen Haupteinsatzbereich für ein erfindungsgemäßes Gehörschutz- und/oder Lautsprecherelement optimal zu gestalten, ist vorgesehen, dass der Tragteil an einem Helm, beispielsweise einem Schutzhelm, montiert oder Teil eines solchen ist.

Auf diese Weise trägt ein Tragteil und ein Modul kaum auf, ist leicht und kann immer sofort bei Bedarf eingesetzt werden.

In diesem Zusammenhang ist natürlich auch die Zugänglichkeit zu den Verstellmöglichkeiten beim Gehörschutz- und/oder Lautsprecherelement von wesentlichem Vorteil. Hier wird vorgeschlagen, dass die Verstelleinrichtung für das Modul an der Außenoberfläche des Helmes bedienbar ist. Vom Benützer selbst kann eine Einstellung bzw. Verstellung bei aufgesetztem Helm durchgeführt werden.

Eine erfindungsgemäße Gehörschutz- und/oder Lautsprecherelement kann aber nach einer anderen Ausführungsvariante auch so ausgebildet sein, dass zwei Tragteile mit entsprechenden Modulen an den Endbereichen eines aufsetzbaren Bogenteil montiert sind. Es ist dann eine Art üblicher Kopfhörer gegeben, wobei dann aber nicht das ganze Ohr des Benützers abdeckende Muscheln vorgesehen werden müssen, sondern beispielsweise eine Art Abstützelemente, so dass auch hier nur das pfropfenartig ausgebildete Modul in Richtung zum Gehörgang zu verstellen ist. Ob nun dieses Modul dann als Gehörschutz oder als Lautsprecher eingesetzt wird oder für beide Funktionen ausgerüstet ist, spielt keine Rolle.

Eine weitere besondere Einsatzmöglichkeit sieht vor, dass der Helm als Fahrzeugsturzhelm ausgeführt ist. Auch hier können in gleicher Art und Weise Gehörschutz-und/oder Lautsprecherelemente vorgesehen werden.

In der nachstehenden Beschreibung werden weitere Einzelheiten der Erfindung anhand der Zeichnung noch näher erläutert. Es zeigen:
- Fig. 1: die Vorderansicht eines Kopfes mit aufgesetztem Schutzhelm;
- Fig. 2: eine Seitenansicht eines Kopfes mit aufgesetztem Schutzhelm;
- Fig. 3: ein vergrößertes Detail aus der Darstellung nach Fig. 1;
- Fig. 4: ein vergrößertes Detail aus der Darstellung nach Fig. 2.

Das erfindungsgemäße Gehörschutz- und/oder Lautsprecheretement wird in der nachstehenden Beschreibung im Zusammenhang mit einem Helm, vorzugweise einem Schutzhelm; erläutert. Es sind grundsätzlich auch andere Möglichkeiten des Einsatzes eines solchen Gehörschutz und/oder Lautsprecherelementes - gegebenenfalls mit einem integrierten Mikrophon - gegeben, die teilweise noch in der nachstehenden Beschreibung angesprochen werden.

Das Gehörschutz und/oder Lautsprecherelementes 1 besteht im Wesentlichen aus einem Tragteil 2 und einem mit einer Ohrmuschel 3 eines Benützers in Wirkverbindung bringbaren, als Gehörschutz und/oder Lautsprecher wirksamen Modul 4. Der Tragteil 2 am Kopf des Benützers festlegbar. Beim gezeigten Beispiel erfolgt dies über einen Schutzhelm 5, an welchem der Tragteil befestigt ist oder gegebenenfalls auch einstückig anschließt. Das als Gehörschutz und/oder Lautsprecher 1 wirksame Modul 4 ist relativ zum Tragteil 2 von einer Ruhestellung in Richtung zu einer Einsatzstellung verstellbar (Pfeilrichtungen 6). Das Modul 4 ist pfropfenartig und gegebenenfalls den Muscheleingang (Gehörgang) eines menschlichen Ohres 3 abdeckender Teil 7 ausgebildet.

Das Modul 4 ist über ein elastisch dehnbares und zusammendrückbares und/oder federnd ausgebildetes Halteelement 8 mit dem Tragteil 2 verbunden. Dabei ist das Modul 4 am Tragteil 2 ausfahrbar und einziehbar gehalten ist.

Am einfachsten ist die in der Zeichnung auch dargestellte Konstruktion. Hier wird das Modul 4 über eine mechanische Verstelleinrichtung mit dem Tragteil 2 verbunden.

Im Rahmen der Erfindung liegt eine vorteilhafte Ausgestaltung darin, das Modul 4 über einen elektrischen Antrieb mit dem Tragteil 2 zu verbinden. Dann können durch Messeinrichtungen oder verschiedene Steuereinrichtungen auch noch vielfältige Möglichkeiten ausgeschöpft werden. Beispielsweise ist dann auch die Verstellung des Moduls 4 gegenüber dem Tragteil 2 in Abhängigkeit von einem voreinstellbaren Lärmpegel und/oder einem eingeschalteten Lautsprecher möglich. Hier können entsprechende Steuerelemente im Schutzhelm 5 integriert sein oder aber es wäre denkbar, von einem in näherer Umgebung befindlichen Messgerät über eine Ansteuerung per Funk das Ausfahren des Moduls zu bewirken.

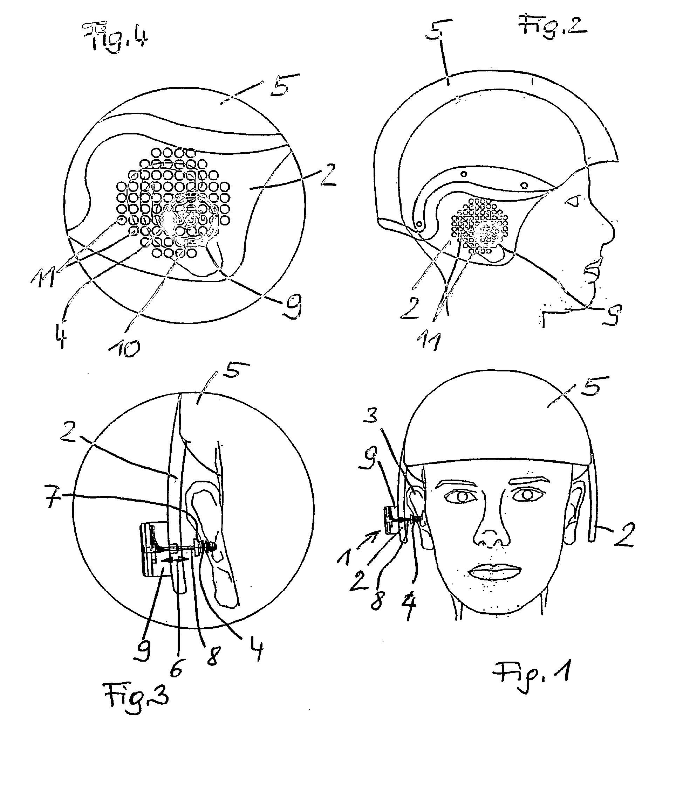

Bei der Darstellung in Fig. 1 ist nur auf einer Seite des Schutzhelmes 5 an dem Tragteil 2 ein Gehörschutz- und/oder Lautsprecherelement 1 angeordnet. Selbstverständlich ist ein gleiches Gehörschutz- und/oder Lautsprecherelement 1 auch an der gegenüberliegenden, vom Betrachter auch rechten Seite vorgesehen. Es sind also zwei Module 4 um 180° versetzt zueinander am gleichen Tragteil 2 angeordnet. Es muss dabei nicht unbedingt der gleiche Tragteil sein, wenn der oder die Tragteile 2 mit dem Schutzhelm 5 verbunden sind.

Besonders vorteilhaft ist die Konstruktion, bei der das Modul 4 über von außerhalb am Tragteil 2 angeordneten Drehknöpfen 9 verstellbar ist. Es können dabei für den Verschiebebereich des Moduls 4 auch Endanschläge vorgesehen sind. Es ist auch möglich, das Modul 4 oder die Verstelleinrichtung in der Vorschubstellung einrastbar auszuführen, damit eine selbsttätige Verstellung infolge von Erschütterungen usw. vermieden werden kann. Trotzdem ist aber dann das Modul 4 elastisch und/oder federnd wegschwenkbar.

Optimal ist die Variante, wenn die Verstelleinrichtung für das Modul 4 an der Außenoberfläche des Helmes bedienbar ist, so dass eine Verstellung möglich ist, ohne jedes Mal den Helm vorher abzunehmen oder anzupassen.

Bei der dargestellten Konstruktion ist das Halteelement 8 als in eine kreis- oder spiralförmig verlaufende Nut 10 im Drehknopf 9 eingelegter, elastisch abbiegbarer Stab ausgeführt ist, welcher im Vorschubbereich des Moduls 4 in die Vorschubrichtung umgelenkt ist. Dieser Stab oder ein entsprechendes Federelement kann im Inneren einen durchgehenden Hohlraum aufweisen, wobei hier Anschlusskabel für den Kopfhörer und/oder ein integriertes Mikrophon zugeführt werden können.

Wie insbesondere den Figuren 2 und 4 entnommen werden kann, ist das Halteelement 8 und somit auch das Modul 4 und somit das gesamte Gehörschutz- und/oder Lautsprecherelement 1 quer zur Vorschubrichtung desselben in vertikaler und/oder horizontaler Richtung verstellbar am Tragteil 2 gehalten sind. Eine hier dargestellt Möglichkeit sieht eine Vielzahl von Löchern 11 vor, in deren Bereich eine Befestigungsmöglichkeit vorhanden ist. Es ist dabei auch denkbar, dass an dem Gehörschutz- und/oder Lautsprecherelement 1 zwei oder noch mehr Zapfen ausgebildet sind, welche in zwei oder mehrer Löcher 11 am Tragteil eingreifen, so dass dadurch auch eine verdrehsichere Montage gewährleistet ist. Je nach der Tragart des Schutzhelmes 5 bei verschiedenen Benutzern und je nach Höhenlage der Ohren oder des Gehörganges kann damit eine individuelle Anpassung erfolgen.

In der Beschreibung ist stets davon ausgegangen worden, dass das Gehörschutz-und/oder Lautsprecherelement 1 im Zusammenhang mit einem Schutzhelm 5 gesehen wird. Der Tragteil 2 ist dann an einem Helm, beispielsweise einem Schutzhelm 5, montiert oder ist gegebenenfalls Teil eines solchen. Es kann ja auch der Schutzhelm 5 möglicherweise einstückig zusammen mit den beiden nach unten abstehenden Tragteilen 2 auszubilden.

Bei einer anderen Variante werden zwei Tragteile 2 mit entsprechenden Modulen 4 an den Endbereichen eines aufsetzbaren Bogenteil montiert. Eine andere Möglichkeit sieht vor, dass der Helm als Fahrzeugsturhelm ausgeführt ist. Gleiche oder ähnliche Voraussetzungen sind auch gegeben und mit der Erfindung lösbar, wenn Fliegerhelme, Motorradhelme oder irgendwelche Sporthelme vorhanden sind.

Im Rahmen der Erfindung sind eine Vielzahl weiterer Einsatzmöglichkeiten für das erfindungsgemäße Gehörschutz- und/oder Lautsprecherelement 1 denkbar. Ein Einsatz ist überall dort möglich, wo in besonderer Weise Gehörschutz erforderlich ist und/oder der Wunsch nach einer unmittelbaren Lautsprecherinformation gegeben ist. Es ist daher auch möglich, verschiedene Arbeitsgruppen über neue Standorte oder über andere Maßnahmen zu orientieren, wobei diese dann nicht über schwere und unhandlich bedienbare Lautsprecher erfolgen muss, sondern es kann eben ein leicht verschiebbares und besonders wirksames Modul 4 eingesetzt werden. Trotzdem kann aber die Verletzungsgefahr ausgeschlossen werden, da das Modul 4 elastisch und federnd nachgiebig aus der Einsatzstellung beim Gehöreingang weggeschwenkt werden kann.

Die konstruktive Gestaltung der Halteelemente 8 und der Verstelleinrichtung kann auf verschiedene Art und Weise erfolgen. Wesentlich und wichtig ist dabei immer, dass eine einfacher und schneller Vorschub des Moduls 4 ermöglicht ist und dass das Modul 4 bei einem Unfall oder bei einer unerwarteten Bewegung des Benützers und somit bei einer möglichen Verschiebung des Schutzhelmes 5 auf dem Kopf des Benützers seitlich wegbewegen kann, ohne das Ohr des Benützers zu verletzen.

Natürlich könnte anstelle des relativ kleinen Moduls 4, welches in den Muscheleingang des Ohres eingreifen kann, auch eine großflächige Abdeckung vorgesehen werden, welche auf die erfindungsgemäße Art und Weise vorgeschoben und zurückgezogen würde, wobei aber dann der wesentliche Vorteil des Freiliegen des gesamten Ohres und somit die Verhinderung der Schweißbildung wegfallen würden.

## Patentansprüche

1. Gehörschutz- und/oder Lautsprecherelement (1) mit einem Tragteil (2) und einem mit einer Ohrmuschel eines Benutzers in Wirkverbindung bringbaren, als Gehörschutz und/oder Lautsprecher wirksamen Modul (4), wobei das Tragteil (2) am Kopf des Benützers festlegbar ist, und das als Gehörschutz und/oder Lautsprecher (1) wirksame Modul (4) relativ zum Tragteil (2) von einer Ruhestellung in Richtung zu einer Einsatzstellung verstellbar ist und wobei das Modul (4) pfropfenartig und gegebenenfalls als ein den Muscheleingang eines menschlichen Ohres abdeckender Teil ausgebildet ist, **dadurch gekennzeichnet, dass** das Modul (4) über ein elastisch dehnbares und zusammendrückbares und/oder federnd ausgebildetes Halteelement (8) mit dem Tragteil (2) verbunden ist, sodass das Modul (4) elastisch und federnd nachgiebig aus einer Einsatzstellung bei einem Gehöreingang des Benützers wegschwenkbar ist.

2. Gehörschutz- und/oder Lautsprecherelement nach Anspruch 1, **dadurch gekennzeichnet, dass** das Modul (4) am Tragteil (2) ausfahrbar und einziehbar gehalten ist.

3. Gehörschutz- und/oder Lautsprecherelement nach den Ansprüchen 1 und/oder 2, **dadurch gekennzeichnet, dass** das Modul (4) über eine mechanische Verstelleinrichtung mit dem Tragteil (2) verbunden ist.

4. Gehörschutz- und/oder Lautsprecherelement nach den Ansprüchen 1 und/oder 2, **dadurch gekennzeichnet, dass** das Modul (4) über einen elektrischen Antrieb mit dem Tragteil (2) verbunden ist.

5. Gehörschutz- und/oder Lautsprecherelement nach einem der Ansprüche 1 bis 4, **dadurch gekennzeichnet, dass** die Verstellung des Moduls (2) gegenüber dem Tragteil (2) in Abhängigkeit von einem voreinstellbaren Lärmpegel und/oder einem eingeschalteten Lautsprecher erfolgt.

6. Gehörschutz- und/oder Lautsprecherelement nach den Ansprüchen 1 bis 5, **dadurch gekennzeichnet, dass** zwei Module (4) um 180° versetzt zueinander am gleichen Tragteil (4) angeordnet sind.

7. Gehörschutz- und/oder Lautsprecherelement nach einem der Ansprüche 1 bis 6, **dadurch gekennzeichnet, dass** das Modul (4) über von außerhalb am Tragteil (2) angeordneten Drehknöpfen (9) verstellbar ist.

8. Gehörschutz- und/oder Lautsprecherelement nach einem der Ansprüche 1 bis 7, **dadurch gekennzeichnet, dass** für den Verschiebebereich des Moduls (4) Endanschläge vorgesehen sind.

9. Gehörschutz- und/oder Lautsprecherelement nach Anspruch 8, **dadurch gekennzeichnet, dass** das Modul (4) oder die Verstelleinrichtung in der Vorschubstellung einrastbar ist, wobei das Modul (4) trotzdem elastisch und/oder federnd wegschwenkbar ist.

10. Gehörschutz- und/oder Lautsprecherelement nach einem der Ansprüche 1 bis 9, **dadurch gekennzeichnet, dass** das Halteelement (8) als in eine kreis- oder spiralförmig verlaufende Nut (10) am Drehknopf (9) eingelegter elastisch abbiegbarer Stab ausgeführt ist, welcher im Vorschubbereich des Moduls (4) in die Vorschubrichtung umgelenkt ist.

11. Gehörschutz- und/oder Lautsprecherelement nach einem der Ansprüche 1 bis 10, **dadurch gekennzeichnet, dass** das Halteelement (8) und somit auch das Modul (4) und somit das gesamte Gehörschutz- und/oder Lautsprecherelement (1) quer zur Vorschubrichtung desselben in vertikaler und/oder horizontaler Richtung verstellbar am Tragteil (2) gehalten sind.

12. Gehörschutz- und/oder Lautsprecherelement nach den vorhergehenden Ansprüchen, **dadurch gekennzeichnet, dass** der Tragteil (2) an einem Helm, beispielsweise einem Schutzhelm (5), montiert oder Teil eines solchen ist.

13. Gehörschutz- und/oder Lautsprecherelement nach Anspruch 12, **dadurch gekennzeichnet, dass** die Verstelleinrichtung für das Modul (5) an der Außenoberfläche des Helmes (5) bedienbar ist.

14. Gehörschutz- und/oder Lautsprecherelement nach einem der Ansprüche 1 bis 13, **dadurch gekennzeichnet, dass** zwei Tragteile (2) mit entsprechenden Modulen (5) an den Endbereichen eines aufsetzbaren Bogenteils montiert sind.

15. Gehörschutz- und/oder Lautsprecherelement nach Anspruch 12, **dadurch gekennzeichnet, dass** der Helm (5) als Fahrzeugsturzhelm ausgeführt ist.

## Claims

1. A hearing protection and/or loudspeaker element (1) having a support part (2) and having a module (4) which can be brought into an operative connection with the external ear of a user and which is operative as hearing protection and/or a loudspeaker, wherein the support part (2) can be secured on the head of the user and the module (4) operative as hearing protection and/or a loudspeaker (1) is movable relative to the support part (2) from an inoperative position towards a use position and wherein the module (4) is plug-like in design and is optionally in the form of a part covering the external-ear entrance of a human ear, **characterised in that** the module (4) is connected to the support part (2) via an elastically extendable and compressible and/or resilient holding element (8), so that the module (4) is elastically and resiliently swingable away from a use position in an ear entrance of the user.

2. A hearing protection and/or loud speaker element according to claim 1, **characterised in that** the module (4) is held on the support part (2) in an extendible and retractable manner.

3. A hearing protection and/or loud speaker element according to claims 1 and/or 2, **characterised in that** the module (4) is connected to the support part (2) via a mechanical adjusting device.

4. A hearing protection and/or loud speaker element according to claims 1 and/or 2, **characterised in that** the module (4) is connected to the support part (2) via an electric drive.

5. A hearing protection and/or loudspeaker element according to any one of claims 1 to 4, **characterised in that** adjustment of the module (2 [sic]) with respect to the support part (2) takes place dependent on a presettable noise level and/or a turned-on loudspeaker.

6. A hearing protection and/or loud speaker element according to claims 1 to 5, **characterised in that** two modules (4) are arranged on the same support part (4[sic]), at 180° to one another.

7. A hearing protection and/or loud speaker element according to any one of claims 1 to 6, **characterised in that** the module (4) is adjustable via turning knobs (9) arranged externally on the support part (2).

8. A hearing protection and/or loud speaker element according to any one of claims 1 to 7, **characterised in that** end stops are provided for the displacement range of the module (4).

9. A hearing protection and/or loud speaker element according to claim 8, **characterised in that** the module (4) or the adjusting device can be locked in the advanced position, the module (4) nonetheless being able to be pivoted away in an elastic and/or resilient manner.

10. A hearing protection and/or loud speaker element according to any one of claims 1 to 9, **characterised in that** the holding element (8) is in the form of an elastically bendable rod placed in a groove (10) running circularly or helically in the turning knob (9), which rod is deflected in the advancing direction, in the advancing range of the module (4).

11. A hearing protection and/or loud speaker element according to any one of claims 1 to 10, **characterised in that** the holding element (8) and consequently the module (4) too and consequently the entire hearing protection and/or loud speaker element (1) are held on the support part (2) so as to be adjustable in a vertical and/or horizontal direction, transversely to the advancing direction of the element (1).

12. A hearing protection and/or loud speaker element according to any one of the preceding claims, **characterised in that** the support part (2) is mounted on a helmet, for example a protective helmet (5), or is a part of the like.

13. A hearing protection and/or loud speaker element according to claim 12, **characterised in that** the adjusting device for the module (5) can be operated at the outer surface of the helmet (5).

14. A hearing protection and/or loud speaker element according to any one of claims 1 to 13, **characterised in that** two support parts (2) with appropriate modules (5[sic]) are mounted on the end regions of an attachable curved part.

15. A hearing protection and/or loud speaker element according to claim 12, **characterised in that** the helmet (5) is in the form of a vehicle crash-helmet.

## Revendications

1. Elément de protection auditive et/ou de haut-parleur (1) comportant un support (2) ainsi qu'un module (4) qui peut être mis en contact avec le pavillon de l'oreille d'un utilisateur comme protection auditive et/ou comme haut-parleur,
le support (2) se fixe sur la tête de l'utilisateur et le module (4) fonctionnant comme protection auditive et/ou comme haut-parleur (1) peut être déplacé par rapport à la partie de support (2) d'une position de repos vers une position d'utilisation, et
le module (4) en forme de bouchon est le cas échéant réalisé comme une pièce couvrant l'entrée du canal auditif de l'oreille,
**caractérisé en ce que**
le module (4) est relié au support (2) par un élément de fixation (8) extensible élastiquement et pouvant être comprimé et/ou élastique, de façon que le module (4) puisse être dégagé d'une position d'utilisation à l'entrée du canal auditif de l'utilisateur par un pivotement élastique et de façon souple.

2. Elément de protection auditive et/ou haut-parleur selon la revendication 1,
**caractérisé en ce que**
le module (4) est relié au support (2) de manière à pouvoir se déployer et se rétracter.

3. Elément de protection auditive et/ou haut-parleur selon les revendications 1 et/ou 2,
**caractérisé en ce que**
le module (4) est relié au support (2) par une installation de réglage mécanique.

4. Elément de protection auditive et/ou haut-parleur selon les revendications 1 et/ou 2,
**caractérisé en ce que**
le module (4) est relié au support (2) par un moteur électrique.

5. Elément de protection auditive et/ou haut-parleur selon l'une des revendications 1 à 4,
**caractérisé en ce que**
le déplacement du module (4) par rapport au support (2) se fait en fonction d'un niveau de bruit préréglé et/ou de l'activation d'un haut-parleur.

6. Elément de protection auditive et/ou haut-parleur selon les revendications 1 à 5,
**caractérisé par**
deux modules (4) décalés de 180° montés sur le même support (4).

7. Elément de protection auditive et/ou haut-parleur selon l'une des revendications 1 à 6,
**caractérisé en ce que**
le module (4) peut être réglé par des boutons de rotation (9) extérieurs au support (2).

8. Elément de protection auditive et/ou haut-parleur selon l'une des revendications 1 à 7,
**caractérisé par**
des butées de fin de course pour la plage de coulissement du module (4).

9. Elément de protection auditive et/ou haut-parleur selon la revendication 8,
**caractérisé en ce que**
le module (4) ou l'installation de réglage peuvent s'encliqueter en position avancée, et néanmoins le module (4) peut être dégagé élastiquement et/ou de façon souple.

10. Elément de protection auditive et/ou haut-parleur selon l'une des revendications 1 à 9,
**caractérisé en ce que**
l'élément de fixation (8) est réalisé sous la forme d'une tige élastique souple insérée dans une rainure en cercle ou en spirale (10) du bouton tournant (9), cette tige étant déviée dans la plage d'avancée du module (4) dans la direction d'avancée.

11. Elément de protection auditive et/ou haut-parleur selon l'une des revendications 1 à 10,
**caractérisé en ce que**
l'élément de fixation (8) et ainsi le module (4) et l'ensemble de l'élément de protection auditive et/ou de haut-parleur (1) sont tenus de façon réglable à la pièce de support (2) par rapport à la direction d'avancée dans la direction verticale et/ou de la direction horizontale.

12. Elément de protection auditive et/ou haut-parleur selon l'une des revendications précédentes,
**caractérisé en ce que**
le support (2) est monté sur un casque, par exemple un casque de protection (5) ou il fait partie d'un tel élément.

13. Elément de protection auditive et/ou haut-parleur selon la revendication 12,
**caractérisé en ce que**
l'installation de réglage du module (5) est desservie au niveau de la surface supérieure extérieure du casque (5).

14. Elément de protection auditive et/ou haut-parleur selon l'une des revendications 1 à 13,
**caractérisé par**
deux supports (2) avec des modules correspondants (5) montés sur les zones d'extrémité d'une partie d'arc de forme rapportée.

15. Elément de protection auditive et/ou haut-parleur selon la revendication 12,
**caractérisé en ce que**
le casque (5) est un casque de pilotage.
